# EUROPÄISCHE PATENTSCHRIFT

(11) **EP 2 926 995 B1**
(45) Veröffentlichungstag und Bekanntmachung des Hinweises auf die Patenterteilung: **22.06.2016**
(21) Anmeldenummer: 15000608.8
(22) Anmeldetag: 03.03.2015
(51) Int. Cl.: B41F 5/24, A61F 13/15, B41J 3/407, B41J 15/16

(54) **VERFAHREN ZUR MODERNISIERUNG EINER BESTEHENDEN SERVIETTENPRODUKTIONSMASCHINE ZUR BEHANDLUNG VON MATERIALBAHNEN UND MODERNISIERTE SERVIETTENPRODUKTIONSMASCHINE**
METHOD FOR MODERNISING AN EXISTING SERVIETTE PRODUCTION MACHINE FOR TREATING SHEETS OF MATERIAL AND MODERNISED SERVIETTE PRODUCTION MACHINE
PROCÉDÉ DE MODERNISATION D'UNE MACHINE DE PRODUCTION DE SERVIETTE EXISTANTE DESTINÉE AU TRAITEMENT DE BANDES DE MATÉRIAU ET MACHINE DE PRODUCTION DE SERVIETTES MODERNISÉE

(30) Priorität: 05.04.2014 DE 102014005032
(43) Veröffentlichungstag der Anmeldung: 07.10.2015
(73) Patentinhaber: SDF Schnitt-Druck-Falz Spezialmaschinen GmbH, 40789 Monheim (DE)
(72) Erfinder: Rother, Andreas, 40780 Monheim (DE); Allard, Peter, 41516 Grevenbroich (DE)

(56) Entgegenhaltungen:
- DE-A1-102009 000 522
- DE-A1-102012 015 838
- GB-A- 409 272

## Beschreibung

Verfahren zur Modernisierung einer bestehenden Serviettenproduktionsmaschine zur Behandlung von Materialbahnen und modernisierte Serviettenproduktionsmaschine

Die Erfindung betrifft ein Verfahren zur Modernisierung einer bestehenden Serviettenproduktionsmaschine zur Behandlung von absorbierenden Materialbahnen für Servietten, insbesondere Tissue- oder Airlaid-Bahnen, wobei die Serviettenproduktionsmaschine zumindest einen Zuführabschnitt, insbesondere eine Abwickelstation umfassend, ein Flexodruckwerk und eine Endbehandlungsstation mit wenigstens einer passerabhängigen Vorrichtung aufweist.

Weiter betrifft die Erfindung eine modernisierte Serviettenproduktionsmaschine Serviettenproduktionsmaschine zur Behandlung einer Materialbahn für Servietten, insbesondere einer Tissue- oder Airlaid-Bahn, mit einem Zuführabschnitt, insbesondere eine Abwickelstation umfassend, einem Flexodruckwerk und einer Endbehandlungsstation mit wenigstens einer passerabhängigen Vorrichtung.

Die Erfindung wird im Folgenden anhand einer Serviettenproduktionsmaschine beschrieben.

Eine derartige Anlage ist dem Fachmann bekannt. Sie umfasst zumindest eine Abwickelstation für Rollen aus Tissue- oder Airlaid-Bahnen, im weiteren Verlauf kurz Bahn genannt. Über einen Bahnlaufpfad wird die Bahn nach dem Abwickeln weitergeführt. Sie passiert dabei verschiedene Stationen bis zur Fertigstellung der fertigen Servietten. So sind verschiedene Leitmittel vorgesehen, die die Bahn markierungsfrei führen. Die Bahn durchläuft zunächst beispielsweise ein Glättwerk, ein Flexodruckwerk zum Bedrucken der Tissue- oder Airlaid-Bahn und anschließend eine Trocknungseinrichtung, die beispielsweise eine Lufttrocknung oder eine Infrarottrocknung sein kann. In dieser Erfindung sei dieser beschriebene Maschinenabschnitt als Zuführabschnitt bezeichnet.

Bereits in der GB-A-409272 sind ein Druckverfahren für Servietten mit bestimmten Farbstoffen und eine Vorrichtung zum Bedrucken von Servietten mit einem oder mehreren Druckzylindern mit elastischer Oberfläche beschrieben.

Bei Anlagen des Standes der Technik folgt auf den Zuführabschnitt in der Regel die Endbehandlungsstation, die verschiedene Einrichtungen umfassen kann. Oft wird die Bahn zumindest durch eine Schneideinrichtung geleitet. Diese kann Schnitte in Bahnlaufrichtung und/oder quer dazu vornehmen. Häufig wird die Bahn auch durch eine Prägestation geführt, die für ein plastisches Aussehen der Bahn sorgen kann. Bei der Herstellung von Servietten müssen Bogen geschnitten und eine Falz- bzw. Faltstation durchlaufen werden. Alle diese in der Anlage vorhandenen passerabhängigen Vorrichtungen werden im Folgenden in Summe als Endbehandlungsstation bezeichnet. Gerade für Falt-, Schneid- oder Prägeeinrichtungen muss es Antriebseinheiten geben, die die Bahn passergenau transportieren. In diesem Fall bezieht sich das Wort "passergenau" bzw. "passerabhängig" nicht nur auf den Farbdruck auf der Bahn sondern auch auf das genaue Schnitt- und Faltmaß zum Druck, sowie die Passergenauigkeit zu einer eventuellen Prägestation.

Ein Stand der Technik hinsichtlich der Aussortierung defekter Hygieneartikel - auch solche aus Tissuebahnen - in einer Verarbeitungsanlage ist aus der US 2010/0305740 A1 bekannt. Diese Anlage weist kein Druckwerk auf.

Das Bedrucken von Nonwowen-Produkten, wie beispielsweise Windeln, beschreiben beispielsweise die US 2005/0116976 A1, die US 2007/0137769 A1 und die US 6092002.

Um die Druckeigenschaften zu verbessern, wird in der DE 10 2012 015 838 A1 eine Bahnbehandlungsanlage vorgeschlagen, die ein digitales

Druckwerk aufweist. Die Flexibilität und Qualität des Drucks auf der absorbierenden Materialbahn wird dadurch deutlich erhöht. Die geeigneten digitalen Druckwerke sind in der DE 10 2012 015 838 A1 ausführlich beschrieben. Eine derartige Anlage ist in ihrer Gesamtheit jedoch sehr kostspielig. Viele Hersteller von Servietten scheuen ein Investitionsrisiko und benutzen weiterhin ihre Behandlungsanlagen, die mit einem herkömmlichen Flexodruckwerk ausgerüstet sind.

Es ist deshalb die Aufgabe der Erfindung, eine bestehende Behandlungsanlage zur Behandlung von absorbierenden Materialbahnen auf kostengünstige Weise zu verbessern.

Die Aufgabe wird hinsichtlich des Verfahrens zur Modernisierung einer bestehenden Anlage zur Behandlung von absorbierenden Materialbahnen durch die Umbau-Verfahrensschritte
- Trennen des Zuführabschnitts von der Endbehandlungsstation,
- Schaffung eines Freiraums zwischen dem Zuführabschnitt und der Endbehandlungsstation,
- Installation eines digitalen Druckwerks innerhalb des Freiraums,
- und Herstellung einer steuerungsmäßigen Verknüpfung der wenigstens einen passerabhängigen Vorrichtung mit der Drucksteuerung des digitalen Druckwerks
gelöst.

Erst mit allen diesen Umbauschritten ist es möglich, beispielsweise eine bestehende Serviettenproduktionsmaschine auf einen digitalen Druck der Tissue- oder Airlaid-Bahn umzustellen. Der einfache Einbau eines nachgeorderten digitalen Druckwerks, wie man es beispielsweise bei der Firma Canon kaufen kann, führt nicht zu einer funktionellen Einheit. Es ist nämlich wichtig, dass der Vorschub innerhalb des digitalen Druckwerks, der durch die eingesetzten Bahnzugsveränderungseinrichtungen regelbar ist, exakt korreliert mit dem Vorschub in der Endbehandlungsstation. Aus diesem Grund wird die wenigstens eine passerabhängige Vorrichtung und die Antriebssteuerung des digitalen Druckwerks steuerungstechnisch miteinander verbunden. Dabei ist hier noch nicht von der Geschwindigkeitssynchronisation die Rede. Es geht durch den Einsatz der zusätzlich installierten Steuerung vornehmlich um die Anpassung der Startpunkte. Mit dem Anlauf der wenigstens einen passerabhängigen Vorrichtung wird also ein Beginn des Drucks eingeleitet. Nur mit dieser Maßnahme ist der nachträgliche Einbau eines digitalen Druckwerk erfindungsgemäß möglich gemacht worden.

Bevorzugt erfolgt über die steuerungsmäßige Verknüpfung ein Startsignal von der wenigstens einen passerabhängigen Vorrichtung (14, 15, 16) an die Drucksteuerung.
Das ist mit handelsüblichen Steuerfahnen oder Drehimpulsgebern in überraschend einfacher Weise realisierbar. Es ergibt sich der Vorteil, dass Druckbilder aus dem digitalen Druckwerk bei einer Bearbeitung in der Endbehandlungsstation immer genau die gleiche Position einnehmen.

Vorteilhaft ist es, wenn die Materialbahn in der Endbehandlungsstation geprägt wird und von einer Prägestation an einem bestimmten Arbeitszeitpunkt das Signal an die Drucksteuerung erfolgt.
Gerade bei Prägestationen zeigt sich, dass ein nachträglicher Einbau eines digitalen Druckwerks in eine bestehende Serviettenproduktionsanlage nicht ohne Weiteres möglich ist. Der entscheidende Punkt ist der Anlauf der beiden Maschinen vom Stillstand aus, wenn also beispielsweise eine neue Rolle in der Abwicklung gestartet wird. Beim Anlauf der beiden Maschinen ist es wichtig, den Druckanfang zu den registerhaltigen Antrieben wie Prägung und Schnitt passergenau übereinander zu bringen. Je schneller dieses erfolgt, desto weniger Ausschuss wird produziert. Aus dem Stand der Technik ist es bekannt, dass bei kompletten Anlagen mit digitalem Druckwerk neben dem Motiv eine Druckmarke gedruckt wird, auf den sich die registerhaltigen Antriebe der passerabhängigen Vorrichtungen über eine Druckmarkensteuerung aufsynchronisieren können. Diese Möglichkeit ist bei dem nachträglichen Einbau eines digitalen Druckwerks in der Regel versagt, da die gebrauchte Maschine häufig nur über einen Zentralantrieb verfügt und insbesondere Positiv-Negativ gravierte Prägewalzen mit der Bahn verzahnt sind. Dementsprechend kann die Umfangsgeschwindigkeit einer Prägewalze nicht langsamer oder schneller als die Geschwindigkeit der Bahn sein, weil es ansonsten zum Einreißen der Bahn kommen würde.

Alternativ oder zusätzlich ist es vorteilhaft, wenn die Materialbahn in der Endbehandlungsstation geschnitten wird und von einer Schneidstation an einem bestimmten Arbeitszeitpunkt das Signal an die Drucksteuerung erfolgt. Hier gilt in ähnlicher Weise das zu der Prägestation Gesagte. Man schneidet hier Bogen gleicher Größe, beispielsweise zur Herstellung von Servietten. Die Servietten werden durch das digitale Druckwerk deutlich aufgewertet, weil sie eine bessere Druckqualität bieten. Außerdem ist eine Individualisierung mit kleineren Stückzahlen einfacher zu realisieren. Alternativ oder zusätzlich ist es bevorzugt, wenn die Materialbahn in der Endbehandlungsstation gefaltet wird. Auch in diesem Fall handelt es sich beispielsweise um Servietten. Mit der Anlagenmodernisierung ist das in dem digitalen Druckwerk erzeugte Motiv genau und immer gleich in den Faltvorgang einzubinden.

In diesen genannten Fällen ist das mit dem Einbau des digitalen Druckwerks entstandene Problem also dadurch gelöst, dass von einem registerhaltigen Antrieb der Prägung oder auch dem des Schnittantriebs ein rapporthaltiges Signal für den Druckanfang erfolgt. Sollte mit dieser Maßnahme noch keine exakte Justierung möglich sein, dient das Signal zuerst einmal der groben Vorsteuerung.

Die Feinjustierung erfolgt dann in vorteilhafter Weise über eine zusätzliche Installation einer Bahnwegveränderungseinrichtung, über die die Länge des Bahnlaufpfades veränderbar ist.
Eine Bahnwegveränderungseinrichtung oder auch bahnwegveränderndes Register genannt, welches mit einem manuellen Stellantrieb oder einem Antriebsmotor ausgestattet sein kann, verändert also die Länge des Bahnlaufpfades. Dadurch können Ungenauigkeiten und Passerversatz ausgeglichen werden. Bei gleicher Längendehnung in der Bahn reicht dieses Verfahren aus, um beim Start und Stopp jeweils Passerhaltigkeit zwischen Druck und Prägung und Schnitt zu erreichen. Zusätzlich kann mit Hilfe einer speziellen Photozelle der Druckanfang innerhalb einer Rapportlänge des digitalen Druckwerks mit dem vom Rapport abhängigen Signal verglichen werden und die Bahnwegveränderungseinrichtung mit einem Antriebsmotor automatisch verstellt werden.
Dabei ist es von Vorteil, wenn die Bahnwegveränderungseinrichtung über eine elektronische Steuerungseinrichtung Signale von einer Antriebseinheit in der Endbehandlungsstation erhält oder an diese abgibt. Diese Signale können einfach ausgewertet werden. So ist es beispielsweise möglich, dass die Signale von einem Antriebsmotor ausgesendet werden und der Vorschub der Antriebseinheit bzw. die Verlängerung oder Verkürzung der Bahnlaufpfadlänge durch die Bahnwegveränderungseinrichtung aufgrund dieser Impulse verändert und anhand einer in der Steuerung vordefinierten Korrelation angepasst wird.

Vorzugsweise wird eine zusätzliche Installation einer Bahnzugveränderungseinrichtung vorgenommen.

Bei Längendehnungsunterschieden gleicht sie den passerhaltigen Versatz aus. Es ist dabei zu beachten, dass eine Tissue- oder Airlaid-Bahn sehr anfällig für Längenänderungen durch die Behandlung in dem digitalen Druckwerk ist. Mit wenigstens einer, bevorzugt aber zwei Bahnzugsveränderungseinrichtungen vor und/oder hinter dem digitalen Druckwerk ist es möglich, eine immer gleichbleibende Motivgröße für die Endbehandlungsvorrichtung zu erzielen.
Weil die Tissue- oder Airlaid-Bahn bei unterschiedlicher Druckbehandlung (bereits bei verschiedenen Druckmotiven hintereinander) im digitalen Druckwerk häufig auch unterschiedliche Längenänderungen aufweist, ist es sinnvoll innerhalb der Steuerung die Bahnspannung im digitalen Druckwerks zu messen, und anhand geeigneter und in der Steuerung hinterlegter Größen über die wenigstens eine Bahnzugveränderungseinrichtung zu regeln. Zur Angleichung der Geschwindigkeiten der Bahn im digitalen Druckwerk und in der Endbehandlungsstation ist es sinnvoll den Antrieb des digitalen Druckwerks als Master einzusetzen und die Antriebseinheit in der Endbehandlungsstation als Slave vorzusehen. Theoretisch ist aber auch eine steuerungstechnisch umgekehrte Zuordnung denkbar. Die Bahnzugveränderungseinrichtung übernimmt letztendlich mit anderen Komponenten die Funktion eines automatischen Längenausdehnungskompensationsregisters, um die Passergenauigkeit einhalten zu können.

Vorzugsweise wird ein vorhandenes Flexodruckwerk als Abgabestation eines Primers an die Materialbahn vor dem digitalen Drucken verwendet. Häufig ist es notwendig, die Tissue- oder Airlaid-Bahn mit einem Primer zu versehen, der ein Eindringen der Tinte aus dem digitalen Druckwerk in das innere der Bahn verhindert. Das Druckbild wäre ansonsten in vielen Fällen nicht zufriedenstellend. Der Primer erfüllt dann die Funktion eines Haftvermittlers für die Farbe. Sofern die modernisierte Anlage zuvor ein Flexodruckwerk besaß, das wegen des neuen digitalen Druckwerks nicht mehr zur farblichen Gestaltung der Tissue- oder Airlaid-Bahn zum Einsatz kommt, wird dieses erfindungsgemäß zu einer "Primerauftragsvorrichtung" umfunktioniert. Über diese lässt sich problemlos eine gewünschte Primerauftragsmenge in Höhe von 0,5 bis 4,5 g/m² aufbringen. Man spart dadurch erhebliche Aufwendungen durch die zusätzliche Anschaffung einer Beschichtungsanlage. Zudem hat die geschaffene "Primerauftragsvorrichtung" den positiven Effekt, Staubpartikel, die sich bei der Behandlung von Tissuebahnen besonders häufig bilden, an der Oberfläche der Bahn zu binden. Außerdem führt dieser Onlinevorgang zu dem weiteren Vorteil, dass eine Beschichtung in einer dem Prozess vorgezogenen Vorbehandlung komplett entfallen kann.

Dabei ist es von Vorteil, wenn die Trennung zwischen der Zuführstation, in der das Flexodruckwerk angeordnet ist, und der Endbehandlungsstation in einem Bereich vorhandener Trocknungseinrichtungen derart erfolgt, dass ein Teil der Trocknungseinrichtungen in Bahnlaufrichtung hinter dem Flexodruckwerk verbleibt und der verbleibende Teil der Trocknungseinrichtungen in Bahnlaufrichtung hinter dem digitalen Druckwerk angeordnet ist. In besonders einfacher Weise kann die ursprüngliche Behandlungsanlage zur Behandlung der Bahn beispielsweise zwischen zwei Trocknungseinrichtungen getrennt werden. In dem entstehenden Freiraum wird das digitale Druckwerk untergebracht. Dann ist es in vorteilhafter Weise möglich, den Primerauftrag aus dem ehemaligen Flexodruckwerk in der ersten Trocknungseinrichtung zu trocknen, und anschließend den Farbauftrag des digitalen Druckwerks mit der zweiten Trocknungseinrichtung trocknen, bevor die Bahn in die Endbehandlung durch Schneiden, Prägen oder Falten geführt wird. Gegebenenfalls sind weitere Trocknungseinrichtungen nachzurüsten.

Die Aufgabe wird hinsichtlich einer modernisierten Serviettenproduktionsmaschine zur Behandlung einer Materialbahn für Servietten, insbesondere einer Tissue- oder Airlaid-Bahn, mit einem Zuführabschnitt, insbesondere eine Abwickelstation umfassend, einem Flexodruckwerk und einer Endbehandlungsstation mit wenigstens einer passerabhängigen Vorrichtung, dadurch gelöst, dass dass eine bestehende Serviettenproduktionsmaschine (1) umgebaut ist, indem
- der Zuführabschnitt (2) von der Endbehandlungsstation (3) getrennt wurde,
- ein Freiraum zwischen dem Zuführabschnitt (2) und der Endbehandlungsstation (3) geschaffen wurde,
- ein digitales Druckwerks (4) innerhalb des Freiraums positioniert wurde,
- und eine steuerungsmäßige Verknüpfung der wenigstens einen passerabhängigen Vorrichtung (14, 15, 16) mit der Drucksteuerung des digitalen Druckwerks (4) hergestellt wurde..

Die übergeordnete Steuerungseinrichtung gibt also ein digitales Signal an einer passerabhängigen Vorrichtung ab, damit das digitale Druckwerk zu einem exakten Zeitpunkt mit dem Druck eines neuen Motivs beginnen kann. Somit ist in der Anlage sichergestellt, dass die Endbehandlungen immer an der gleichen Position des wiederkehrenden Druckbildes beginnen. Ein leichtes Abbremsen der Bahn in der Endbehandlungsstation kann über eine Bahnzugveränderungseinrichtung ausgeglichen werden. Eine zweite Bahnzugveränderungseinrichtung kann beispielsweise bei der faltenfreien Durchführung der Bahn durch das digitale Druckwerk behilflich sein und die Spannungen in der Bahn im Druckwerk konstant halten.

Mit Vorteil ist vorgesehen, dass hinter dem digitalen Druckwerk eine Bahnwegveränderungseinrichtung vorgesehen ist.

Die Vorteile sind bereits bei dem analogen abhängigen Verfahrensanspruch erläutert worden.

Bevorzugt ist die Ausgestaltung der Bahnwegveränderungseinrichtung so, dass die Längenänderung des Bahnlaufpfades durch eine Verstellbarkeit einer von der Bahn umschlungenen Walze ermöglicht ist. Die Verstellung der Walze erfolgt dabei im Wesentlichen quer zur Bahnlaufrichtung. Die Walze dringt in den Bahnlaufpfad ein und verlängert dabei den Weg der Bahn. Die Bewegung der Walze in der Bahnwegveränderungseinrichtung kann durch Stellmotoren oder Schwenkhebel erfolgen.

Bevorzugt ist vor und/oder hinter dem digitalen Druckwerk eine Bahnzugveränderungseinrichtung angeordnet. Eine zweite Bahnzugveränderungseinrichtung kann beispielsweise bei der faltenfreien Durchführung der Bahn durch das digitale Druckwerk behilflich sein. Bahnzugsveränderungseinrichtungen sind bekannt. Dabei bilden beispielsweise zwei Walzen einen Nip, über den auf die Bahn ein Zug aufgebracht werden kann. Aber vielfach wird der gleiche Zweck auch durch die Reibung an umschlungenen Walzen erreicht.
Dabei ist es von Vorteil, wenn wenigstens eine Bahnzugveränderungseinrichtung zwei von der Bahn S-förmig umschlungene Walzen umfasst. Durch die große Umschlingung ist ein Bahnzug auf besonders einfache Weise veränderbar, wenn wenigstens eine der Walzen angetrieben ist. Bevorzugt sind jedoch beide Walzen mit einem separaten Motor angetrieben, um mehr Einflussmöglichkeiten auf die Bahnspannung und den Vorschub ausüben zu können.
Bevorzugt besitzt wenigstens eine der Walzen eine Oberfläche mit einem Haftreibungskoeffizienten zu der Bahn größer 0,6.
Durch eine hohe Haftreibung µ_{H} an der Walzenoberfläche kann mittels des Antriebsmotors aufgrund der Reibung eine höhere Zugspannung auf die Bahn aufgebracht werden. Vielfach werden die Walzen deshalb mit einem Bezug, beispielsweise aus einem aufgetragenem Molybdänbelag oder Epoxydharz, versehen. Die Umschlingung der Walze liegt bevorzugt bei über 60°. Diese Werte haben sich in Versuchsreihen als besonders vorteilhaft herausgestellt.

Vorzugsweise ist zwischen einer Antriebseinheit in der Endbehandlungsstation und dem Antrieb des digitalen Druckwerks eine Master-Slave-Steuerung oder -regelung zur Geschwindigkeitssynchronisation vorgesehen. Die Steuerung wird dadurch erheblich vereinfacht, weil nur auf die Vorgabe entweder der Antriebseinheit in der Endbehandlungsstation oder des Druckwerks eine Regelung des Bahnvorschubs erfolgen muss.

Vorzugsweise weist die modernisierte Serviettenproduktionsmaschine neben dem digitalen Druckwerk ein Flexodruckwerk auf. Dabei ist das Flexodruckwerk bereits vor der Modernisierung vorhanden gewesen. Das Flexodruckwerk lässt sich dann mit Vorteil, wie bereits beschrieben, derart umrüsten, dass es geeignet ist, einen Primer vor dem digitalen Druckvorgang an die Materialbahn abzugeben.

Wie bereits im Verfahren zur Modernisierung erläutert, ist es günstig, wenn in der Serviettenproduktionsmaschine sowohl vor als auch hinter dem digitalen Druckwerk Trocknungseinrichtungen vorgesehen sind. Die Bahn kann dann sowohl nach der Beaufschlagung mit dem Primer als auch nach dem digitalen Druck getrocknet werden.

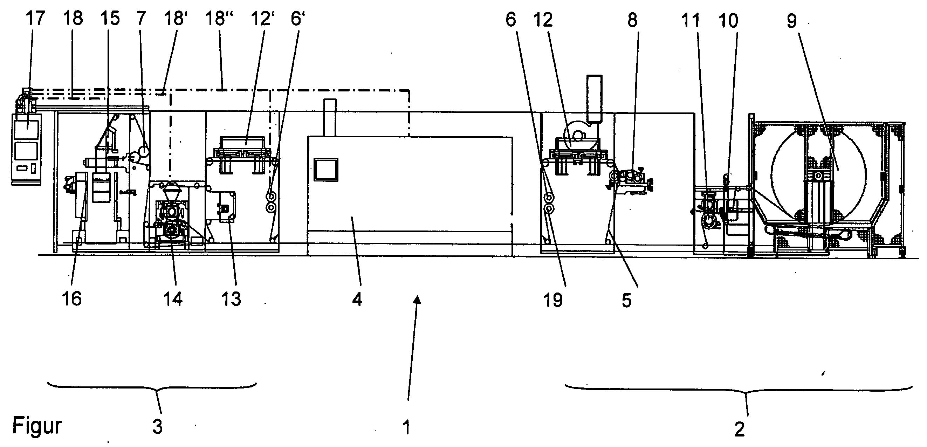

Die Erfindung wird im Folgenden anhand eines Ausführungsbeispiels unter Bezugnahme auf die Zeichnung näher erläutert.
Die Figur zeigt dabei eine modernisierte Serviettenproduktionsmaschine 1 zur Behandlung einer Warenbahn 5, insbesondere einer Tissue- oder Airlaid-Bahn.

Auf der rechten Seite der Figur ist der erste Teil der ehemaligen Serviettenproduktionsmaschine dargestellt, der Zuführabschnitt 2. In Warenbahnlaufrichtung umfasst dieser Zuführabschnitt 2 eine Abwicklung 9 in Form einer Drehsternabrollung, eine Staubabsaugung 10, um die für einen Druckvorgang störenden freien Tissuefasern zu entfernen, einen Satinierkalander 11, um die Bahn vor den Druck zu glätten, ein Flexodruckwerk 8, auf das später eingegangen wird, und eine erste Trocknungseinrichtung 12 der Bahn.

Links erkennt man den ehemaligen zweiten Teil, den Endbehandlungsabschnitt 3. Dieser umfasst eine weitere Trocknungseinrichtung 12'. Es folgen die weiteren Aggregate zur Endbehandlung, die hohe Anforderungen an die Passergenauigkeit stellen. Zunächst folgt nach der Bahnkantensteuerung 13 ein Prägewerk 14, mit dem der Bahn 5 eine Struktur oder ein Muster aufgegeben wird. Schließlich wird die Endbehandlungsstation 3 erweitert um eine Falzstation 15 und eine Schneid- und Faltstation 16. Für die Erfindung sind verständlicherweise nicht alle drei Aggregate notwendig. Es genügt, wenn die Endbehandlungsstation eines der Vorrichtungen 14, 15, 16 aufweist, das registermäßig zu dem Druckbild passen muss.

Um die Längen dieser Abschnitte zu verdeutlichen, ist in der Figur eine geschweifte Klammer den Bezugszeichen 2 und 3 zugeordnet. Die ehemalige Anlage, in der der Zuführabschnitt 2 und der Endbehandlungsabschnitt 3 unmittelbar aneinander grenzten, wird geteilt. Zwischen dem Zuführabschnitt 2 und dem Endbehandlungsabschnitt 3 wird ein Freiraum geschaffen, in dem ein digitales Druckwerk 4 untergebracht ist. Mit dem Bezugszeichen 22 ist der Bahnlaufpfad der Bahn 5 bezeichnet.

Das digitale Druckwerk 4 ermöglicht eine höhere Qualität bei dem Bedrucken der Servietten als das herkömmliche Flexodruckwerk. Zudem sind höhere Individualisierungen möglich, die einen Einsatz des digitalen Druckwerks innerhalb eines akzeptablen Zeitraums rentiert.

Die Nachrüstung der bestehenden Behandlungsanlage mit einem Druckwerk war allerdings mit bislang ungelösten Problemen verbunden. So ist die Massenträgheit der vorhandenen Anlage, also der Serviettenmaschine, wesentlich größer ist als die des Druckwerks, so dass es zu einem Aufschwingen der Antriebe kommt. Zudem bewirkt die unterschiedliche Längenausdehnung des Tissues bei unterschiedlichen Motiven bzw. unterschiedlichen Farbaufträgen, dass eine Synchronisation mit Prägung und Schnitt bislang unmöglich erschien. Außerdem wurden extreme Probleme mit Feuchtigkeitsunterschieden in der Bahn 5 erwartet.

Erfindungsgemäß sind diese Probleme gelöst worden. Dazu wird eine Signalabgabe von einem Antrieb 7 einer passerabhängigen Vorrichtung 14, 15, 16 an die Steuerung des Druckwerks ermöglicht. Über den Schnitt oder die Faltung oder die Prägung der Bahn 5 wird dem Druckwerk auf diese Weise vorgegeben, wann ein neuer Druck zu beginnen hat. Hier sei darauf verwiesen, dass die Darstellung des Antriebs 7 nur aus Gründen der Übersichtlichkeit an der gezeigten Stelle dargestellt ist. Es kann sich in der Realität um einen Antrieb für ein Prägewerk 14, eine Falzstation 15 oder eine Schneid- und Faltstation 16 handeln.

Damit ist die Übereinstimmung der Passer bereits sehr genau. Sollten Feinjustierungen notwendig werden, so geschieht dies über eine Bahnwegveränderungseinrichtung 21. Die Walze 23 der Bahnwegveränderungseinrichtung 21 kann manuell verstellt oder über Antriebe in die Bahn eintauchen (angedeutet über den Doppelpfeil) und somit deren Bahnlaufpfad verlängern oder verkürzen.

Dehnungen der Bahn 5 durch Feuchtigkeit oder Belastung im Druckwerk 4 werden durch Bahnzugveränderungseinrichtung 6, 6' vor und/ oder nach dem digitalen Druckwerk 4 kompensiert. Die Bahnspannungen werden dazu über Bahnspannungsmesswalzen 20, 20' ermittelt

Mehrere Vorrichtungen 14, 15, 16, 21, 6, 6', 20, 20' sind über Steuerleitungen 18, 18', 18" mit einer gemeinsamen Steuereinrichtung 17 verbunden. Von dieser werden Steuerungssignale über die Steuerleitungen 18, 18' an die Antriebseinheiten 7 des Prägewerks 14, der Falzstation 15 oder der Schneid- und Faltstation 16 weitergegeben. Die Vorgaben für die Geschwindigkeitssynchronisation erfolgen von dem Druckwerk an die Steuerung 17. Die Regelung verläuft also nach dem Master-Slave-Prinzip.

In dem Ausführungsbeispiel sind sowohl vor als auch hinter dem digitalen Druckwerk 4 Bahnzugveränderungseinrichtungen 6, 6' installiert. Damit lässt sich auch die Bahnspannung und somit die Dehnung im digitalen Druckwerk 4 beeinflussen. Die Bahnzugveränderungseinrichtungen 6, 6' ist realisiert durch zwei angetriebene, S-förmig umschlungene Walzen 19, die keinen Kontakt zueinander haben, um die Bahn nicht zu beschädigen. Es wird dafür gesorgt, dass die Reibung in dem oder den Umschlingungswinkel(n), die möglichst mehr als 60° betragen, so groß ist, dass ein Bahnzug durch winzige Veränderungen in der Antriebsdrehzahl aufgebracht werden kann. Dafür ist vorgesehen, dass wenigstens eine der Walzen eine Oberfläche mit einem Haftreibungskoeffizienten zu der Bahn größer 0,6 besitzt. Dies ist beispielsweise mit dem Auftrag einer geeigneten Oberflächenbeschichtung möglich.

Zur Verbesserung des Druckbildes, das im digitalen Druckwerk erzeugt wird, ist vorgesehen, dass das nicht mehr genutzte Flexodruckwerk 8 umgerüstet wird, Primer auf die Bahn 5 aufzutragen. Im digitalen Druckwerk ist die Bahn dann quasi beschichtet, so dass die aufgetragene Farbe nicht mehr so tief in die Tissue- oder Airlaid-Bahn eindringt und stattdessen, wie gewünscht, an der Oberfläche verbleibt.

In besonders günstiger Weise wurde dazu die Trennung zwischen dem Zuführabschnitt 2 und der Endbehandlungsstation 3 genau zwischen zwei ehemals vorhandenen Trocknungseinrichtungen 12, 12' vorgenommen. So kann die in Bahnlaufrichtung erste Trocknungseinrichtungen 12 die Bahn nach dem Primerauftrag und die zweite Trocknungseinrichtungen 12' die Bahn nach dem Farbauftrag im digitalen Druckwerk 4 trocknen. Sollte bei der zu modernisierenden Behandlungsanlage nur eine, nicht trennbare Trocknungseinrichtungen vorhanden sein, müsste in diesem Fall eine zweite zusätzlich mit in dem Freiraum zwischen dem Zuführabschnitt 2 und der Endbehandlungsstation 3 verbaut werden.

### Bezugszeichenliste

- 1: Anlage zur Behandlung von Materialbahnen
- 2: Zuführabschnitt
- 3: Endbehandlungsstation
- 4: digitales Druckwerk
- 5: Bahn; Tissue- oder Airlaid-Bahn
- 6, 6': Bahnzugveränderungseinrichtung
- 7: Antriebseinheit
- 8: Flexodruckwerk
- 9: Abwicklung, Drehsternabrollung
- 10: Staubabsaugung
- 11: Satinierkalander
- 12, 12': Trocknungseinrichtung
- 13: Bahnkantensteuerung
- 14: Prägewerk
- 15: Falzstation
- 16: Schneid- und Faltstation
- 17: Steuerungseinrichtung
- 18, 18', 18": Signalleitung, Steuerleitung
- 19: Walze
- 20, 20': Bahnzugmesswalze
- 21: Bahnwegveränderungseinrichtung
- 22: Bahnlaufpfad
- 23: Walze der Bahnwegveränderungseinrichtung

## Patentansprüche

1. Verfahren zur Modernisierung einer bestehenden Serviettenproduktionsmaschine zur Behandlung von absorbierenden Materialbahnen für Servietten (5), insbesondere Tissue- oder Airlaid-Bahnen, mit einem Bahnlaufpfad (22), wobei die Serviettenproduktionsmaschine zumindest einen Zuführabschnitt (2), insbesondere eine Abwickelstation (9) umfassend, ein Flexodruckwerk (8) und eine Endbehandlungsstation (3) mit wenigstens einer passerabhängigen Vorrichtung (14, 15, 16) aufweist,
**gekennzeichnet durch** folgende Umbau-Verfahrensschritte:
- Trennen des Zuführabschnitts (2) von der Endbehandlungsstation (3),
- Schaffung eines Freiraums zwischen dem Zuführabschnitt (2) und der Endbehandlungsstation (3),
- Installation eines digitalen Druckwerks (4) innerhalb des Freiraums,
- und Herstellung einer steuerungsmäßigen Verknüpfung der wenigstens einen passerabhängigen Vorrichtung (14, 15, 16) mit der Drucksteuerung des digitalen Druckwerks (4).

2. Verfahren gemäß Anspruch 1, **dadurch gekennzeichnet, dass** über die steuerungsmäßige Verknüpfung ein Startsignal von der wenigstens einen passerabhängigen Vorrichtung (14, 15, 16) an die Drucksteuerung erfolgt.

3. Verfahren gemäß Anspruch 2, **dadurch gekennzeichnet, dass** die Materialbahn (5) in der Endbehandlungsstation (3) geprägt wird und von einer Prägestation (14) an einem bestimmten Arbeitszeitpunkt das Signal an die Drucksteuerung erfolgt.

4. Verfahren gemäß Anspruch 2, **dadurch gekennzeichnet, dass** die Materialbahn (5) in der Endbehandlungsstation (3) geschnitten wird und von einer Schneidstation (16) an einem bestimmten Arbeitszeitpunkt das Signal an die Drucksteuerung erfolgt.

5. Verfahren gemäß einem der Ansprüche 1 bis 4, **dadurch gekennzeichnet, dass** eine zusätzliche Installation einer Bahnwegveränderungseinrichtung (21), über die die Länge des Bahnlaufpfades (22) veränderbar ist, vorgenommen wird.

6. Verfahren gemäß einem der Ansprüche 1 bis 5, **dadurch gekennzeichnet, dass** eine zusätzliche Installation einer Bahnzugveränderungseinrichtung (6, 6') vorgenommen wird.

7. Verfahren gemäß einem der Ansprüche 1 bis 6, **dadurch gekennzeichnet, dass** ein vorhandenes Flexodruckwerk (8) als Abgabestation eines Primers an die Materialbahn (5) vor dem digitalen Drucken verwendet wird.

8. Verfahren gemäß Anspruch 7, **dadurch gekennzeichnet, dass** die Trennung zwischen der Zuführstation (2), in der das Flexodruckwerk (8) angeordnet ist, und der Endbehandlungsstation (3) in einem Bereich vorhandener Trocknungseinrichtungen (12, 12') derart erfolgt, dass ein Teil der Trocknungseinrichtungen (12) in Bahnlaufrichtung hinter dem Flexodruckwerk (8) verbleibt und der verbleibende Teil der Trocknungseinrichtungen (12') in Bahnlaufrichtung hinter dem digitalen Druckwerk (4) angeordnet ist.

9. Modernisierte Serviettenproduktionsmaschine (1) zur Behandlung einer Materialbahn für Servietten (5), insbesondere einer Tissue- oder Airlaid-Bahn, mit einem Zuführabschnitt (2), insbesondere eine Abwickelstation (9) umfassend, einem Flexodruckwerk (8) und einer Endbehandlungsstation (3) mit wenigstens einer passerabhängigen Vorrichtung (14, 15, 16) und einer elektronischen Steuerungseinrichtung (17), die geeignet ist, startsignale von einer passerabhängigen vorrichtung (14, 15, 16) in der Endbehandlungsstation (3) an die Steuerung des digitalen Druckwerks (4) abzugeben, **dadurch gekennzeichnet, dass** eine bestehende Serviettenproduktionsmaschine (1) umgebaut ist, indem
- der Zuführabschnitt (2) von der Endbehandlungsstation (3) getrennt wurde,
- ein Freiraum zwischen dem Zuführabschnitt (2) und der Endbehandlungsstation (3) geschaffen wurde,
- ein digitales Druckwerks (4) innerhalb des Freiraums positioniert wurde,
- und eine steuerungsmäßige Verknüpfung der wenigstens einen passerabhängigen Vorrichtung (14, 15, 16) mit der Drucksteuerung des digitalen Druckwerks (4) hergestellt wurde..

10. Modernisierte Serviettenproduktionsmaschine gemäß Anspruch 9, **dadurch gekennzeichnet, dass** hinter dem digitalen Druckwerk (4) eine Bahnwegveränderungseinrichtung (21) vorgesehen ist.

11. Modernisierte Serviettenproduktionsmaschine gemäß Anspruch 10, **dadurch gekennzeichnet, dass** die Bahnwegveränderungseinrichtung (21) die Längenänderung des Bahnlaufpfades (22) durch eine Verstellbarkeit einer von der Bahn (5) umschlungenen Walze (23) ermöglicht.

12. Modernisierte Serviettenproduktionsmaschine gemäß einem der Ansprüche 9 bis 11, **dadurch gekennzeichnet, dass** vor und/oder hinter dem digitalen Druckwerk (4) eine Bahnzugveränderungseinrichtung (6, 6') angeordnet ist.

13. Modernisierte Serviettenproduktionsmaschine gemäß einem der Ansprüche 9 bis 12, **dadurch gekennzeichnet, dass** zwischen einer Antriebseinheit (7) in der Endbehandlungsstation (3) und dem Antrieb des Druckwerks (4) eine Master-Slave-Steuerung oder -regelung zur Geschwindigkeitssynchronisation vorgesehen ist.

14. Modernisierte Serviettenproduktionsmaschine gemäß einem der Ansprüche 9 bis 13, **dadurch gekennzeichnet, dass** sie neben dem digitalen Druckwerk (4) ein Flexodruckwerk (8) aufweist, wobei das Flexodruckwerk(8) geeignet ist, einen Primer vor dem digitalen Druckvorgang an die Materialbahn (5) abzugeben.

15. Modernisierte Serviettenproduktionsmaschine gemäß einem der Ansprüche 9 bis 14, **dadurch gekennzeichnet, dass** sowohl vor als auch hinter dem digitalen Druckwerk (4) Trocknungseinrichtungen (12, 12') vorgesehen sind.

## Claims

1. Method for modernizing an existing serviette production machine for treating absorbent sheets of material for serviettes (5), in particular tissue webs or air-laid webs, with a sheet running path (22), wherein the serviette production machine has at least one feeding section (2), in particular comprising an unwinding station (9), a flexographic printing unit (8) and a final treatment station (3) with at least one register-dependent device (14, 15, 16),
**characterized by** the following converting method steps:
- separating the feeding section (2) from the final treatment station (3),
- creating a clearance between the feeding section (2) and the final treatment station (3),
- installing a digital printing unit (4) within the clearance,
- and establishing a control link from the at least one register-dependent device (14, 15, 16) to the printing controller of the digital printing unit (4).

2. Method according to Claim 1, **characterized in that** a starting signal of the at least one register-dependent device (14, 15, 16) to the printing controller takes place by way of the control link.

3. Method according to Claim 2, **characterized in that** the sheet of material (5) is embossed in the final treatment station (3) and, at a certain working time, the signal to the printing controller takes place from an embossing station (14).

4. Method according to Claim 2, **characterized in that** the sheet of material (5) is cut in the final treatment station (3) and, at a certain working time, the signal to the printing controller takes place from a cutting station (16).

5. Method according to one of Claims 1 to 4, **characterized in that** an additional installation of a sheet-path changing device (21), by way of which the length of the sheet running path (22) is variable, is performed.

6. Method according to one of Claims 1 to 5, **characterized in that** an additional installation of a sheet-tension changing device (6, 6') is performed.

7. Method according to one of Claims 1 to 6, **characterized in that** an existing flexographic printing unit (8) is used as a discharging station for a primer onto the sheet of material (5) before the digital printing.

8. Method according to Claim 7, **characterized in that** the separation between the feeding station (2), in which the flexographic printing unit (8) is arranged, and the final treatment station (3) takes place in a region of existing drying devices (12, 12'), **in that** part of the drying devices (12) remains downstream of the flexographic printing unit (8) in the sheet running direction and the remaining part of the drying devices (12') is arranged downstream of the digital printing unit (4) in the sheet running direction.

9. Modernized serviette production machine (1) for treating a sheet of material for serviettes (5), in particular a tissue web or air-laid web, with a feeding section (2), in particular comprising an unwinding station (9), a flexographic printing unit (8) and a final treatment station (3) with at least one register-dependent device (14, 15, 16), and an electronic control device (17), which is suitable for emitting starting signals from a register-dependent device (14, 15, 16) in the final treatment station (3) to the controller of the digital printing unit (4), **characterized in that** an existing serviette production machine (1) has been converted, **in that**
- the feeding section (2) has been separated from the final treatment station (3),
- a clearance between the feeding section (2) and the final treatment station (3) has been created,
- a digital printing unit (4) has been positioned within the clearance,
- and a control link from the at least one register-dependent device (14, 15, 16) to the printing controller of the digital printing unit (4) has been established.

10. Modernized serviette production machine according to Claim 9, **characterized in that** a sheet-path changing device (21) is provided downstream of the digital printing unit (4).

11. Modernized serviette production machine according to Claim 10, **characterized in that** the sheet-path changing device (21) allows changing of the length of the sheet running path (22) by an adjustability of a roller (23) around which the sheet (5) passes.

12. Modernized serviette production machine according to one of Claims 9 to 11, **characterized in that** a sheet-tension changing device (6, 6') is arranged upstream and/or downstream of the digital printing unit (4).

13. Modernized serviette production machine according to one of Claims 9 to 12, **characterized in that** a master-slave open-loop or closed-loop control for speed synchronization is provided between a drive unit (7) in the final treatment station (3) and the drive of the printing unit (4).

14. Modernized serviette production machine according to one of Claims 9 to 13, **characterized in that**, apart from the digital printing unit (4), it has a flexographic printing unit (8), wherein the flexographic printing unit (8) is suitable for discharging a primer onto the sheet of material (5) before the digital printing operation.

15. Modernized serviette production machine according to one of Claims 9 to 14, **characterized in that** drying devices (12, 12') are provided both upstream and downstream of the digital printing unit (4).

## Revendications

1. Procédé de modernisation d'une machine de production de serviettes existante servant au traitement de bandes de matière absorbante pour des serviettes (5), notamment des bandes de papier de soie ou d'airlaid, comprenant un chemin de défilement de bande (22), la machine de production de serviettes possédant au moins une portion d'acheminement (2), comprenant notamment un poste de déroulage (9), un groupe de flexographie (8) et un poste de finition (3) pourvu d'au moins un dispositif (14, 15, 16) dépendant du repère,
**caractérisé par** les étapes de transformation suivantes :
- séparation de la portion d'acheminement (2) du poste de finition (3),
- création d'un espace libre entre la portion d'acheminement (2) et le poste de finition (3),
- installation d'un groupe d'impression numérique (4) à l'intérieur de l'espace libre,
- et établissement d'une liaison fonctionnelle de commande de l'au moins un dispositif (14, 15, 16) dépendant du repère avec la commande d'impression du groupe d'impression numérique (4).

2. Procédé selon la revendication 1, **caractérisé en ce qu'**une signalisation de départ de l'au moins un dispositif (14, 15, 16) dépendant du repère à la commande d'impression est effectuée par le biais de la liaison fonctionnelle de commande.

3. Procédé selon la revendication 2, **caractérisé en ce que** la bande de matière (5) est gaufrée dans le poste de finition (3) et la signalisation à la commande d'impression est effectuée par un poste de gaufrage (14) à un instant de travail donné.

4. Procédé selon la revendication 2, **caractérisé en ce que** la bande de matière (5) est coupée dans le poste de finition (3) et la signalisation à la commande d'impression est effectuée par un poste de coupe (16) à un instant de travail donné.

5. Procédé selon l'une des revendications 1 à 4, **caractérisé par** la réalisation d'une installation supplémentaire d'un système de modification de la course de la bande (21), lequel permet de modifier la longueur du chemin de défilement de bande (22).

6. Procédé selon l'une des revendications 1 à 5, **caractérisé par** la réalisation d'une installation supplémentaire d'un système de modification de la traction de la bande (6, 6').

7. Procédé selon l'une des revendications 1 à 6, **caractérisé en ce qu'**un groupe de flexographie (8) existant est utilisé comme poste de distribution d'un promoteur d'adhésion à la bande de matière (5) avant l'impression numérique.

8. Procédé selon la revendication 7, **caractérisé en ce que** la séparation entre le poste d'acheminement (2), dans lequel est disposé le groupe de flexographie (8), et le poste de finition (3) s'effectue dans une zone de systèmes de séchage (12, 12') existants de telle sorte qu'une partie des systèmes de séchage (12) demeure après le groupe de flexographie (8) dans le sens de défilement de la bande, et la partie restante des systèmes de séchage (12') est disposée après le groupe d'impression numérique (4) dans le sens de défilement de la bande.

9. Machine de production de serviettes (1) modernisée servant au traitement d'une bande de matière pour des serviettes (5), notamment une bande de papier de soie ou d'airlaid, possédant une portion d'acheminement (2), comprenant notamment un poste de déroulage (9), un groupe de flexographie (8) et un poste de finition (3) pourvu d'au moins un dispositif (14, 15, 16) dépendant du repère, et un système de commande électronique (17) qui est adapté pour délivrer des signaux de départ d'un dispositif (14, 15, 16) dépendant du repère dans le poste de finition (3) à la commande du groupe d'impression numérique (4), **caractérisée en ce qu'**une machine de production de serviettes (1) existante est transformée **en ce que**
- la portion d'acheminement (2) a été séparée du poste de finition (3),
- un espace libre a été créé entre la portion d'acheminement (2) et le poste de finition (3),
- un groupe d'impression numérique (4) a été positionné à l'intérieur de l'espace libre,
- et une liaison fonctionnelle de commande de l'au moins un dispositif (14, 15, 16) dépendant du repère avec la commande d'impression du groupe d'impression numérique (4) a été établie.

10. Machine de production de serviettes modernisée selon la revendication 9, **caractérisée en ce qu'**un système de modification de la course de la bande (21) est prévu après le groupe d'impression numérique (4).

11. Machine de production de serviettes modernisée selon la revendication 10, **caractérisée en ce que** le système de modification de la course de la bande (21) permet la modification de la longueur du chemin de défilement de bande (22) par une possibilité de positionnement d'un cylindre (23) autour duquel s'enroule la bande (5).

12. Machine de production de serviettes modernisée selon l'une des revendications 9 à 11, **caractérisée en ce qu'**un système de modification de la traction de la bande (6, 6') est disposé avant et/ou après le groupe d'impression numérique (4).

13. Machine de production de serviettes modernisée selon l'une des revendications 9 à 12, **caractérisée en ce qu'**une commande ou une régulation de type maître/esclave servant à la synchronisation de vitesse est prévue entre une unité d'entraînement (7) dans le poste de finition (3) et le mécanisme d'entraînement du groupe d'impression (4).

14. Machine de production de serviettes modernisée selon l'une des revendications 9 à 13, **caractérisée en ce qu'**outre le groupe d'impression numérique (4), elle possède un groupe de flexographie (8), le groupe de flexographie (8) étant adapté pour délivrer un promoteur d'adhésion à la bande de matière (5) avant l'impression numérique.

15. Machine de production de serviettes modernisée selon l'une des revendications 9 à 14, **caractérisée en ce que** des systèmes de séchage (12, 12') sont prévus à la fois avant et après le groupe d'impression numérique (4).
